Europäisches Patentamt

European Patent Office

Office européen des brevets

(19)

(11) Publication number: **0 064 830**

**B1**

(12) # EUROPEAN PATENT SPECIFICATION

(45) Date of publication of patent specification: **26.02.86**

(21) Application number: **82302120.9**

(22) Date of filing: **26.04.82**

(51) Int. Cl.⁴: **A 61 K 7/04**, A 61 K 9/08,
A 61 K 31/415

(54) **Pharmaceutical compositions for treating nail infections.**

(30) Priority: **29.04.81 GB 8113291**

(43) Date of publication of application:
**17.11.82 Bulletin 82/46**

(45) Publication of the grant of the patent:
**26.02.86 Bulletin 86/09**

(84) Designated Contracting States:
**AT BE CH DE FR GB IT LI LU NL SE**

(56) References cited:
**DE-A-1 923 336
FR-A-1 365 298
FR-A-1 538 155
GB-A- 801 726**

**CHEMICAL ABSTRACTS; vol. 82, no. 18, 5th
May 1975, page 281, no. 116003a, Columbus
Ohio (USA)**

**SOAP, PERFUMERY AND COSMETICS, vol. 48,
no. 7, July 1975, pages 299-301, United Trade
Press Ltd, London (GB); "Foot ailments and
products for the feet".**

(73) Proprietor: **Pfizer Limited
Ramsgate Road
Sandwich Kent CT13 9NJ (GB)**

(84) **GB**

(73) Proprietor: **Pfizer Corporation
Calle 15 1/2 Avenida Santa Isabel
Colon (PA)**

(84) **BE CH DE FR IT LI LU NL SE AT**

(72) Inventor: **Davison, Edward
45 Leicester Avenue
Cliftonville Margate Kent (GB)**

(74) Representative: **Moore, James William
Pfizer Limited Ramsgate Road
Sandwich Kent CT13 9NJ (GB)**

(56) References cited:
**CHEMICAL ABSTRACTS, vol. 90, Part 7, 12th
February 1979, page 58, no. 48569c, Columbus
Ohio (USA); S. WAHAB et al.: "Comparative in
vitro and in vivo evaluation of Tolciclate,
tonlaftate, miconazole, clotrimazole and
undecylenic acid against Trichophyton
mentagrophytes".**

Courier Press, Leamington Spa, England.

# 0 064 830

## Description

This invention relates to pharmaceutical compositions and in particular to compositions for treating fungal infections of the nails and surrounding tissues.

Treatment of nail disorders due to fungal infections in the past has been notoriously difficult being both lengthy and often prone to failure. The most common conditions may be classified as chronic paronychia, where the finger nails and nail folds are invaded by Candida species; ringworm infections of the nail plate (usually the toe-nails and often associated with tinea pedis), and non-dermatophyte mould infections. The organisms implicated are the dermatophytes, most commonly Trichophyton rubrum or Trichophyton interdigitalis; moulds, such as Aspergillus versicolor; and yeasts, Candida albicans and Candida parapsilosis being the two species most commonly found.

A variety of treatments have been used in the past but in recent years the newer broad-spectrum imidazole antifungal agents such as clotrimazole, miconazole and tioconazole have been advocated. The proven clinical efficacy of these agents has led to their widespread acceptance for the topical treatment of fungal infections. However treatment of nail infections remains protracted and somewhat uncertain due to the difficulty of penetrating the horny nail plate to reach the site of infection which is frequently in the nail bed and the inner layers of the nail.

We have now discovered that compositions may be prepared containing a high concentration of the imidazole antifungal agent in undecylenic acid and these are able to facilitate penetration of the antifungal agent through the nail-plate. Moreover the composition is readily applied to the nail and surrounding tissues where the antifungal agent remains in liquid solution and is therefore better able to penetrate the nail plate to reach the site of infection.

Thus according to the present invention, there is provided a pharmaceutical composition for the treatment of fungal nail infections which comprises a solution of an imidazole antifungal agent in undecylenic acid, wherein said imidazole antifungal agent is present in a proportion of from 40% to 60% by weight of the mixture of imidazole antifungal agent and undecylenic acid.

The imidazole antifungal agents are well known as a class, they are characterized by possessing an imidazole ring as part of their chemical structure and include such agents as clotrimazole, miconazole, econazole, tioconazole and isoconazole. Tioconazole is particularly preferred because of its broad spectrum and high potency.

Generally the proportion of imidazole antifungal agent to fatty acid within the above-specified range will be determined by the solubility of the particular agent in undecylenic acid up to a practical maximum proportion of a 1:1 molar ratio of the two compounds. We have discovered that solutions containing over 50% by weight of the imidazole in undecylenic acid can easily be achieved. In practice a proportion of 40—60% by weight of the imidazole antifungal agent in undecylenic acid is used as such solutions remain liquid on application and, because of the high concentration of the antifungal agent, only a thin film is needed to provide effective therapy.

The compositions of the invention may be applied in a number of ways. They are most simply brushed or painted onto the affected areas or they may be applied using a felt-tip applicator or cotton wool pad. For ease of application the solution of the imidazole and the fatty solvent is best diluted with a non-toxic volatile organic solvent. This reduces the viscosity of the composition and assists application and spread of the composition onto the affected regions. After application, however, the solvent evaporates leaving the composition of the antifungal agent and the fatty acid as a film on the surface of the nail plate and surrounding tissues. Suitable solvents for this application are those pharmaceutically acceptable organic solvents in wich the mixture of the imidazole and the fatty acid is soluble and which have a sufficiently low boiling point to ensure their rapid evaporation after application. Examples are lower alkanols such as ethanol and isopropyl alcohol, esters such as ethyl acetate and ketones such as acetone and 2-butanone; ethyl acetate and 2-butanone being preferred solvents. The amount of volatile solvent required in any particular case will depend upon the nature of the solvent and the components of the composition and may vary from 10 to 90% by weight of the total formulation. Normally a proportion of 40 to 60% by weight of solvent in the composition will be sufficient to produce a solution having the desired mobility.

Other components may be added to the compositions if desired, for example perfumes, colouring or antioxidants.

The compositions of the invention have advantages over simple solutions of the imidazole antifungal agent in volatile solvents which dry to produce a hard glassy film on the surface of the nail, or over conventional formulations, which do not produce high concentrations of the antifungal agent on the nail or facilitate penetration.

The ability of the compositions of the invention to penetrate nail tissue is assessed using an apparatus consisting of a cell in which two compartments are separated by a nail disc cut from human toe nails. The nail disc is 1 cm. in diameter and is mounted between two teflon rings leaving a surface area of 0.71 cm$^2$ exposed. The cell is vacuum tested for leaks before use. The formulation containing the imidazole antifungal agent is placed in one compartment and methanol in the other. The cell is rotated at a constant 30 revolutions per minute and samples of the methanol are removed from time to time and assayed for presence of the antifungal agent, using both bioassay and radiolabelling techniques. At the end of 30 days

2

the experiment is terminated and the nail disc sectioned using a microtome. Each section is examined and the distribution of the antifungal agent throughout the nail recorded.

Results of such experiments show that formulations of tioconazole and undecylenic acid are particularly effective, good levels of the antifungal agent being observed both in the methanol solution and throughout the nail disc.

The following Examples are provided to further illustrate the invention.

Example 1

Tioconazole (0.56 g) was added to undecylenic acid B.P. (0.44 g) and the mixture gently warmed and agitated until all the tioconazole had dissolved. The mixture was cooled to yield a clear solution. No separation was observed even after prolonged storage at 4°C.

Example 2

The procedure of Example 1 was followed but using the following imidazole antifungal agents and undecylenic acid. In each case a clear solution was obtained on cooling.

| | Proportion in Composition (g) | |
|---|---|---|
| Antifungal agent | Imidazole | Undecylenic acid |
| Miconazole | 0.60 | 0.44 |
| Clotrimazole | 0.50 | 0.44 |
| Econazole | 0.40 | 0.44 |

Example 3

The procedure of Example 1 was followed but using the following proportions of tioconazole and undecylenic acid. In each case a clear solution was obtained on cooling.

| Proportion in composition (% by weight) | |
|---|---|
| Tioconazole | Undecylenic acid |
| 50 | 50 |
| 60 | 40 |

Example 4

The product of Example 1 (0.5 g) was diluted with ethyl acetate U.S.N.F. (0.5 g) to give a clear solution.

Example 5

Tioconazole (0.28 g) and undecylenic acid B.P. (0.22 g) were added to ethyl acetate U.S.N.F. (0.5 g) to give a clear solution identical to the product of Example 4.

Example 6

The procedure of Example 5 is followed but using the following volatile solvents instead of ethyl acetate:

Acetone
Ethanol
Isopropyl alcohol
Butan-2-one

Example 7

The procedure of Example 5 is followed but using the following proportions of active ingredients and volatile solvents:

| Tioconazole (g) | Undecylenic acid (g) | Ethyl acetate (g) |
|---|---|---|
| 0.28 | 0.22 | 5.0 |
| 0.28 | 0.22 | 1.25 |
| 2.8 | 2.2 | 2.2 |
| 2.8 | 2.2 | 0.55 |

Example 8

A solution was prepared by the procedure of Example 5 having the following composition:

| | Parts/100 g |
|---|---|
| Tioconazole | 28 |
| Undecylenic acid | 22 |
| Butan-2-one | 50 |
| Butylated hydroxy toluene BP | 0.1 |

The solution was filtered and filled into dark glass bottles (10 ml) which were fitted with a cap and applicator brush.

**Claims for the Contracting States: BE, CH, DE, FR, GB, IT, LI, LU, NL, SE**

1. A pharmaceutical composition for the treatment of fungal nail infections which comprises a solution of an imidazole antifungal agent in undecylenic acid wherein said imidazole antifungal agent is present in a proportion of from 40% to 60% by weight of the mixture of imidazole antifungal agent and undecylenic acid.

2. A composition as claimed in claim 1 wherein the imidazole antifungal agent is tioconazole.

3. A composition as claimed in any preceding claim which further contains a non-toxic volatile organic solvent.

4. A composition as claimed in claim 3 wherein the volatile organic solvent is ethyl acetate or 2-butanone.

5. A composition as claimed in claim 3 or 4 wherein the volatile organic solvent is present in an amount of from 40% to 60% of the weight of the composition.

6. A composition as claimed in claim 1 wherein the imidazole antifungal agent is tioconazole and wherein the mixture is diluted with ethyl acetate or 2-butanone in an amount of from 40 to 60% of the total weight of the composition.

**Claims for the Contracting State: AT**

1. A process for preparing a pharmaceutical composition for the treatment of fungal nail infections which comprises dissolving an imidazole antifungal agent in undecylenic acid, wherein said imidazole antifungal agent is present in a proportion of from 40% to 60% by weight of the mixture of imidazole antifungal agent and undecylenic acid.

2. A process as claimed in claim 1 wherein the imidazole antifungal agent is tioconazole.

3. A process as claimed in any preceding claim which further comprises diluting the mixture of the imidazole antifungal agent and undecylenic acid with a non-toxic volatile organic solvent.

4. A process as claimed in claim 3 wherein the volatile organic solvent is ethyl acetate or 2-butanone.

5. A process as claimed in claim 3 or 4 wherein the volatile organic solvent is present in an amount of from 40% to 60% of the weight of the composition.

6. A process as claimed in claim 1 wherein the imidazole antifungal agent is tioconazole and wherein the mixture is diluted with ethyl acetate or 2-butanone in an amount of from 40 to 60% of the total weight of the composition.

4

# 0 064 830

**Patentansprüche für die Vertragsstaaten: BE, CH, DE, FR, GB, IT, LI, LU, NL, SE**

1. Pharmazeutische Zusammensetzung für die Behandlung von Nagelpilzinfektionen, welche eine Lösung eines Imidazol-Antipilzmittels in Undecylensäure umfaßt, worin das genannte Imidazol-Antipilzmittel in einem Anteil von 40 bis 60 Gew.% der Mischung von Imidazol-Antipilzmittel und Undecylensäure vorhanden ist.

2. Zusammensetzung, wie in Anspruch 1 beansprucht, worin das Imidazol-Antipilzmittel Tioconazol ist.

3. Zusammensetzung, wie in einem vorhergehenden Anspruch beansprucht, welche ein nichttoxisches flüchtiges organisches Lösungsmittel enthält.

4. Zusammensetzung, wie in Anspruch 3 beansprucht, worin das flüchtige organische Lösungsmittel Äthylacetat oder 2-Butanon ist.

5. Zusammensetzung, wie in Anspruch 3 oder 4 beansprucht, worin das flüchtige organische Lösungsmittel in einem Anteil von 40 bis 60% des Gewichtes der Zusammensetzung vorhanden ist.

6. Zusammensetzung, wie in Anspruch 1 beansprucht, worin das Imidazol-Antipilzmittel Thioconazol ist und worin die Mischung mit Äthylacetat oder 2-Butanon in einem Anteil von 40 bis 60% des Gesamtgewichtes der Zusammensetzung verdünnt ist.

**Patentansprüche für den Vertragsstaat Österreich:**

1. Verfahren zum Herstellen· einer pharmazeutischen Zusammensetzung zur Behandlung von Nagelpilzinfektionen, welches das Lösen eines Imidazol-Antipilzmittels in Undecylensäure umfaßt, wobei das genannte Imidazol-Antipilzmittel in einem Anteil von 40 bis 60 Gew.% der Mischung von Imidazol-Antipilzmittel und Undecylensäure vorhanden ist.

2. Verfahren, wie in Anspruch 1 beansprucht, worin das Imidazol-Antipilzmittel Tioconazol ist.

3. Verfahren, wie in einem vorhergehenden Anspruch beansprucht, welches weiters das Verdünnen der Mischung des Imidazol-Antipilzmittels und von Undecylensäure mit einem nicht-toxischen flüchtigen organischen Lösungsmittel umfaßt.

4. Verfahren, wie in Anspruch 3 beansprucht, worin das flüchtige organische Lösungsmittel Äthylacetat oder 2-Butanon ist.

5. Verfahren, wie in Anspruch 3 oder 4 beansprucht, worin das flüchtige organische Lösungsmittel in einem Anteil von 40 bis 60% des Gewichtes der Zusammensetzung vorhanden ist.

6. Verfahren, wie in Anspruch 1 beansprucht, worin das Imidazol-Antipilzmittel Tioconazol ist und worin die Mischung mit Äthylacetat oder 2-Butanon in einem Anteil von 40 bis 60%, bezogen auf das Gesamtgewicht der Zusammensetzung, verdünnt wird.

**Revendications pour les Etats Contractants: BE, CH, DE, FR, GB, IT, LI, LU, NL, SE**

1. Composition pharmaceutique destinée au traitement d'infections fongiques des ongles, qui comprend une solution d'un agent antifongique imidazolique dans l'acide undécylénique, dans laquelle l'agent antifongique imidazolique est présent en une proportion de 40 à 60% en poids du mélange d'agent antifongique imidazolique et d'acide undécylénique.

2. Composition suivant la revendication 1, dans laquelle l'agent antifongique imidazolique est le tioconazole.

3. Composition suivant l'une quelconque des revendications précédentes, qui contient en outre un solvant organique volatil non toxique.

4. Composition suivant la revendication 3, dans laquelle le solvant organique volatil est l'acétate d'éthyle ou la 2-butanone.

5. Composition suivant la revendication 3 ou 4, dans laquelle le solvant organique volatil est présent en une quantité de 40 à 60% du poids de la composition.

6. Composition suivant la revendication 1, dans laquelle l'agent antifongique imidazolique est le tioconazole et le mélange est dilué à l'acétate d'éthyle ou à la 2-butanone en une quantité de 40 à 60% du poids total de la composition.

**Revendications pour l'Etat Contractant: AT**

1. Procédé de préparation d'une composition pharmaceutique destinée au traitement d'infections fongiques des ongles, qui consiste à dissoudre un agent antifongique imidazolique dans de l'acide undécylénique, l'agent antifongique imidazolique étant présent en une proportion de 40 à 60% en poids du mélange d'agent antifongique imidazolique et d'acide undécylénique.

2. Procédé suivant la revendication 1, dans lequel l'agent antifongique imidazolique est le tioconazole.

3. Procédé suivant l'une quelconque des revendications précédentes, qui consiste en outre à diluer le mélange de l'agent antifongique imidazolique et d'acide undécylénique avec un solvant organique volatil non toxique.

4. Procédé suivant la revendication 3, dans lequel le solvant organique volatil est l'acétate d'éthyle ou la 2-butanone.

5

5. Procédé suivant la revendication 3 ou 4, dans lequel le solvant organique volatil est présent en une quantité de 40 à 60% du poids de la composition.

6. Procédé suivant la revendication 1, dans lequel l'agent antifongique imidazolique est le tioconazole et le mélange est dilué à l'acétate d'éthyle ou à la 2-butanone en une quantité de 40 à 60% du poids total de la composition.